# EUROPEAN PATENT SPECIFICATION

(11) **EP 3 226 853 B1**
(45) Date of publication and mention of the grant of the patent: **04.09.2019**
(21) Application number: 15816590.2
(22) Date of filing: 01.12.2015
(51) Int. Cl.: A61K 31/14, A61K 31/46, A61P 27/02

(54) **ANIMAL MODEL FOR DRY EYE AND METHODS OF USE OF SUCH ANIMALS**
TIERMODELL FÜR DAS SYNDROM DES TROCKENEN AUGES UND VERWENDUNG VON SOLCHEN TIEREN
MODÈLE ANIMAL POUR LA SÉCHERESSE OCULAIRE ET L'UTILISATION DE TELS ANIMAUX

(30) Priority: 02.12.2014 US 201462086263 P
(43) Date of publication of application: 11.10.2017
(73) Proprietor: Regeneron Pharmaceuticals, Inc., Tarrytown, NY 10591-6707 (US)
(72) Inventor: YUAN, Ming, Tarrytown, New York 10591-6707 (US); HU, Ying, Tarrytown, New York 10591-6707 (US); CAO, Jingtai, Tarrytown, New York 10591-6707 (US); ROMANO, Carmelo, Tarrytown, New York 10591-6707 (US)
(74) Representative: Hutter, Anton
(86) International application number: PCT/US2015/063084
(87) International publication number: WO 2016/089807

(56) References cited:
- POOJA JAIN ET AL: "An NGF mimetic, MIM-D3, stimulates conjunctival cell glycoconjugate secretion and demonstrates therapeutic efficacy in a rat model of dry eye", EXPERIMENTAL EYE RESEARCH, ACADEMIC PRESS LTD, LONDON, vol. 93, no. 4, 19 June 2011 (2011-06-19), pages 503-512, XP028331572, ISSN: 0014-4835, DOI: 10.1016/J.EXER.2011.06.014 [retrieved on 2011-06-25]
- SABRINA VIAU ET AL: "No consequences of dietary n-3 polyunsaturated fatty acid deficiency on the severity of scopolamine-induced dry eye", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 249, no. 4, 16 December 2010 (2010-12-16), pages 547-557, XP019893290, ISSN: 1435-702X, DOI: 10.1007/S00417-010-1576-6
- BEYAZYILDIZ E ET AL: "Efficacy of topical mesenchymal stem cell therapy in the treatment of experimental dry eye syndrome model", STEM CELLS INTERNATIONAL 2014 HINDAWI PUBLISHING CORPORATION USA, vol. 2014, 17 July 2014 (2014-07-17), XP002753756, ISSN: 1687-9678
- ZHANG ZHEN ET AL: "Therapeutic Effects of Topical Doxycycline in a Benzalkonium Chloride-Induced Mouse Dry Eye Model", IOVS, vol. 55, no. 5, May 2014 (2014-05), pages 2963-2974, XP002753757,
- LIN ZHIRONG ET AL: "Serine Protease Inhibitor A3K Suppressed the Formation of Ocular Surface Squamous Metaplasia in a Mouse Model of Experimental Dry Eye", IOVS, vol. 55, no. 9, September 2014 (2014-09), pages 5813-5820, XP002753758,

## Description

### BACKGROUND OF THE DISCLOSURE

Dry eye disease (DED), or keratoconjunctivitis sicca (KCS), is described in the 2007 report of the Dry Eye Workshop (DEWS) as a "multifactorial disease of the tears and ocular surface that results in symptoms of discomfort, visual disturbance, and tear film instability with potential damage to the ocular surface which is accompanied by increased osmolarity of the tear film and inflammation of the ocular surface." (Ocular Surf 5(2):75-92, 2007). It has been estimated that about 3.23 million women and 1.68 million men, for a total of 4.91 million Americans, 50 years and older have dry eye. Tens of millions more have less severe symptoms and probably a more episodic manifestation of the disease *(Id.).*

There are two generally recognized subgroups of dry eye, namely, dry eye associated with aqueous deficiency/ reduced tear production, and dry eye associated with increased tear film evaporation ("evaporative dry eye").

Among the aqueous deficient group, there are two major subclasses: Sjögren's syndrome (SS) dry eye and non-SS dry eye. In Sjogren syndrome, the lacrimal glands are targeted by a systemic autoimmune process. The lacrimal glands are infiltrated by activated T-cells, which cause acinar and ductular cell death and hyposecretion of the tears. Several autoimmune diseases are associated with SS dry eye syndrome, such as rheumatoid arthritis, scleroderma, polymyositis, lymphoma, amyloidosis, hemochromatosis, sarcoidosis, and systemic lupus erythematosus (Djalilian AR, et al. Dry eye. In: Krachmer JH, Mannis MJ, Holland EJ, editors. Cornea. 2nd ed. Philadelphia. Elsevier Mosby; 2005). Non-Sjogren syndrome dry eye is a form of aqueous deficiency resulting from lacrimal dysfunction, where the systemic autoimmune features of SS dry eye have been excluded. The most common form of non-SS dry eye is age-related dry eye (Ocular Surf 5(2):75-92, 2007).

Evaporative dry eye results from increased water loss from the ocular surface despite normal lacrimal secretory function. Its causes have been categorized as intrinsic, such as resulting from disease affecting lid structures or dynamics, or extrinsic, where ocular surface disease occurs due to some extrinsic exposure, such as harsh environmental conditions.

An altered hormonal state (e.g., following menopause) can lead to development or exacerbation of dry eye. Several other external factors are also known to cause or contribute to dry eye, such as contact lens wear, refractive laser surgery, smoking, and extended visual tasks such as computer use, watching television and prolonged reading. Worsening of dry eye is also associated with low humidity conditions found in office environments, air-conditioned cars, and extreme hot or cold weather. Dry eye may also be caused by medications such as antihistamines, antidepressants, antipsychotics, and diuretics, which decrease tear production (Ocular Surf 5(2):75-92, 2007).

Patients with dry eye present with conjunctival inflammation manifested by T cell infiltrates and upregulation of CD3, CD4, and CD8, as well as lymphocyte activation markers CD11a and HLA-DR (Stern ME, et al. Invest Ophthalmol Vis Sci. 43:2609-2614 (2002)). Thus, dry eye pathogenesis may be dependent on T-cell activation and autoimmune inflammation. Pro-inflammatory cytokines, such as interleukin (IL)-1, and matrix metalloproteinases (MMPs), have also been implicated in the pathogenesis of dry eye. An increase in the pro-inflammatory forms of IL-1 (IL-1α and mature IL-1β) and a decrease in the biologically inactive precursor IL-1β have been found in the tear film of dry eye patients (Solomon A, et al. Invest Ophthalmol Vis Sci. 42:2283-2292 (2001)).

Pooja et al. ("An NGF mimetic, MIM-D3, stimulates conjunctival cell glycoconjugate secretion and demonstrates therapeutic efficacy in a rat model of dry eye", Experimental Eye Research, col. 93, no. 4, pages 503-512) evaluates the efficacy of MIM-D3, a small molecule nerve growth factor (NGF) peptidomimetic, as a therapeutic agent in rats with scopolamine induced dry eye.

Viau et al. ("No consequences of dietary n-3 polyunsaturated fatty acid deficiency on the severity of scopolamine-induced dry eye", Graefe's archive for clinical and experimental ophthalmology, vol. 294, no. 4, pages 547-557) evaluates whether a dietary deficiency in n-3 polyunsaturated fatty acids (PUFAs) increases the severity of the pathology in a scopolamine-induced model of dry eye in the rat.

Beyazyildiz et al. ("Efficacy of Topical Mesenchymal Stem Cell Therapy in the Treatment of Experimental Dry Eye Syndrome Model", Stem Cells International, vol. 2014, Article ID 250230) considered the efficacy of topically applied mesenchymal stem cells (MSCs) on dry eye syndrome (DES) induced by benzalkonium chloride (BAC) in rats.

Zhang et al. ("Therapeutic effects of topical doxycycline in a benzalkonium chloride-induced mouse dry eye model", Investigative Ophthalmology & Visual Science, vol. 55, no. 5, pages 2963-2974) investigated the therapeutic effects and underlying mechanisms of topical doxycycline in a benzalkonium chloride (BAC)-induced mouse dry eye model.

Lin et al. ("Serine protease inhibitor A3K suppressed the formation of ocular surface squamous metaplasia in a mouse model of experimental dry eye", Investigative Ophthalmology & Visual Science, vol. 55, no. 9, pages 5813-5820) investigated the effects and possible mechanisms of serine protease inhibitor A3K (SERPINA3K) on the formation of ocular surface squamous metaplasia in a mouse dry eye model induced by topical benzalkonium chloride (BAC).

### BRIEF SUMMARY OF THE DISCLOSURE

This disclosure relates to an animal model of dry eye disease. Disclosed herein are methods of inducing dry eye disease in a rodent animal that reflects the pathophysiology of dry eye disease (DED) in humans. The rodent animal can be a mouse or rat. Further disclosed are methods of use of the rodent animals with induced dry eye disease, for example, in testing candidate agents for the treatment of DED.

The methods disclosed herein induce DED by administration of both scopolamine and benzalkonium chloride (BAC) to a rodent animal.

Scopolamine can be administered by various means, including systemic administration such as injection, transdermal patch, and implantation of a pump into the rodent. For example, scopolamine can be administered by an implantation of a pump to provide infusion at a concentration of 0.4-4.0 mg per 20 g. body weight per day. Benzalkonium chloride can be administered topically to the ocular surface of the rodent. For example, BAC can be administered at a concentration of 0.05-0.4%, one to four times per day.

Dry eye condition is characterized in rodent animals treated with scopolamine and BAC by reduced tear production and increased ocular irritation, relative to control levels (e.g., an eye in untreated rodents). In one example, reduced tear production is measured by detecting a reduction in tear production relative to control levels. In another example, increased ocular irritation is measured by detecting, within the eye, one or more of: an increase in inflammatory cells, an increase in inflammatory cytokines in the eye, or an increase in fluorescein staining, relative to control levels. By "reduction" and "increase", it is meant a difference of at least 10%, 20%, 30%, 40%, 50%, 75%, 100% or more, relative to control levels.

Upon administration of scopolamine and BAC to a rodent animal, dry eye condition is evident within days of the administration, e.g., within 5, 7, 10, 14, 21 or 28 days of the administration. In accordance with the methods disclosed herein, administration of scopolamine and BAC can continue for one or more weeks, such as two, three or four weeks, and up to two months or longer if desired.

Further disclosed herein are methods of testing the efficacy of a candidate agent for the treatment of dry eye. The methods involve providing a rodent animal, administering scopolamine and benzalkonium chloride to the rodent animal to induce dry eye in the rodent animal, administering a candidate agent to the rodent animal with dry eye, and determining whether the candidate agent is effective in treating dry eye in the rodent animal.

In some embodiments, the candidate agent is administered at a time point after the start of administration of scopolamine and benzalkonium chloride, such that the efficacy of the candidate agent to ameliorate the symptoms or conditions of DED can be evaluated. For example, the candidate agent can be administered 3, 4, 5, 6, 7, 14, or 28 days after the start of administration of scopolamine and benzalkonium chloride. The efficacy of the candidate agent can be determined by measuring an increase in tear production and/or a decrease in ocular irritation, relative to control levels (e.g., eyes in animals receiving scopolamine and benzalkonium chloride but not receiving the candidate agent).

In some embodiments, the candidate agent is administered concurrently with the start of administration of scopolamine and benzalkonium chloride. In these embodiments, the ability of a candidate agent to prevent DED, e.g., to prevent or delay the onset, or limit the development, of dry eye, can be evaluated. The efficacy of the candidate agent can be determined by measuring the ability of the candidate agent to limit or eliminate reduction in tear production, and/or limit or eliminate increased ocular irritation, relative to control levels (e.g., eyes in animals receiving scopolamine and benzalkonium chloride but not receiving the candidate agent).

The candidate agent can be administered topically or systemically, which may depend on the nature of the candidate agent. Examples of topical administration include administration of the candidate agent in a drop, spray, or gel form to the eye or nose of the rodent animal. Examples of systemic administration include oral administration, injection, or infusion.

### BRIEF DESCRIPTION OF THE FIGURES

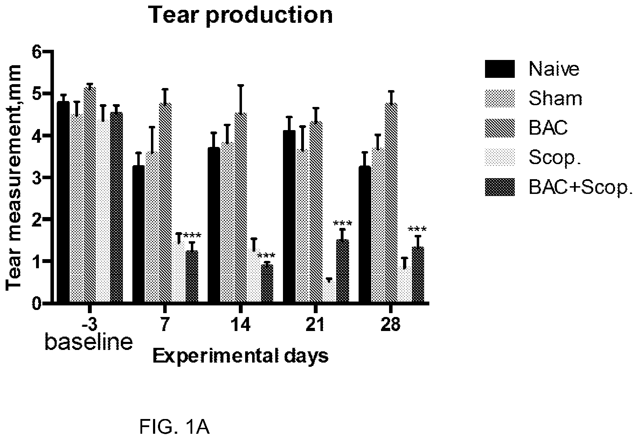

FIGS 1A-1B. (A), represents the measurement of tear production either before initiation of treatment (day -3, baseline) or on days 7, 14, 21, and 28 after initiation of treatment; on each day, the five bars represent measurements from left to right for: naive, sham, BAC, Scop., and BAC+Scop. treatments. Tear production significantly decreases over four weeks of administration of scopolamine (Scop) and administration of benzalkonium chloride plus scopolamine (BAC + Scop), relative to controls or BAC treatment alone. *** *=p*<*0.001.* (B), represents measurement of corneal fluorescein stain score either before initiation of treatment (day -3, baseline) or on days 7, 14, 21, and 28 after initiation of treatment; one each day, the five bars represent measurements from left to right for: naive, sham, BAC, Scop., and BAC+Scop. treatments. Corneal fluorescein staining significantly increases over four weeks of administration of BAC + Scop, relative to controls and relative to treatment with either BAC or Scop alone. **** = *p*<*0.0001*.

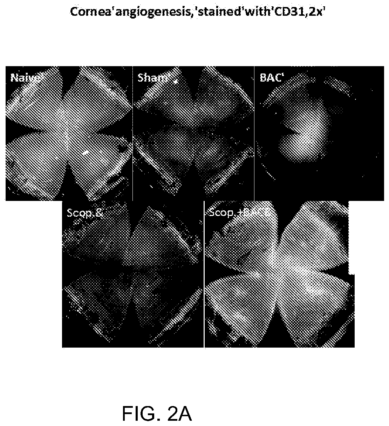

FIGS. 2A-2D. (A), corneal angiogenesis, as measured by increased CD31+ cell staining, increases in BAC + Scop treated eyes, relative to controls and relative to treatment with either BAC or Scop alone. (B), corneal blood vessel skeletonization (new blood vessel) length increases dramatically by four weeks' treatment in BAC + Scop treated eyes, relative to controls and relative to treatment with either BAC or Scop alone. (C), lymphangiogenesis, as measured by increased LYVE1 cell staining, increases in BAC + Scop treated eyes, relative to controls and relative to treatment with either BAC or Scop alone. (D), corneal lymphatic vessel skeletonization length increases dramatically by four weeks' treatment n BAC + Scop treated eyes, relative to controls and relative to treatment with either BAC or Scop alone.

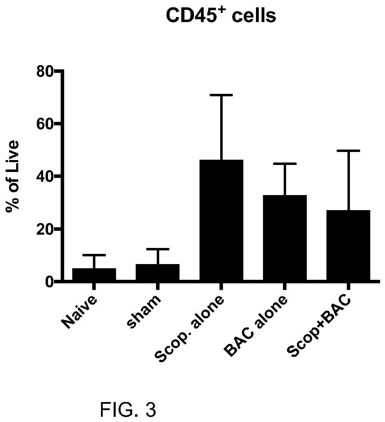

FIG. 3 Flow cytometry of extraorbital lacrimal glands. Percent of CD45+ cells is increased in cells treated with Scop, BAC, and Scop + BAC, relative to controls.

### DETAILED DESCRIPTION OF THE DISCLOSURE

Disclosed herein are methods of inducing dry eye disease in an animal that reflects the pathophysiology of dry eye disease (DED) in humans. Further disclosed are methods of use of the rodent animals with induced dry eye disease in testing candidate agents for the treatment of DED.

Dry eye disease (DED), also known as Dry eye syndrome (DES) or keratoconjunctivitis sicca (KCS), is a condition characterized physiologically by one or more, two or more, three or more, or all of: reduced tear production, increased tear film evaporation, ocular inflammation, increased osmolarity (salt content) of the tears, and damage to the ocular surface. Symptoms of DED include ocular discomfort, visual disturbance, and tear film instability.

Disclosed herein are methods of inducing dry eye disease in a rodent animal. The methods involve administration of scopolamine, an anticholinergic agent, and benzalkonium chloride (BAC), an ocular irritant, to a rodent animal, in an amount and for a time sufficient to induce dry eye disease in the animal. The resulting ocular disorder displays multiple characteristics of human DED.

In developing the approach disclosed herein, the inventors were able to induce dry eye disease in rodent animals that closely resembles the clinical human disease. For example, the dry eye conditions induced with the disclosed methods are easier to perform and can last for weeks. Further, the animal models produced by the disclosed methods display both the ocular inflammation and tear deficiency commonly found in human dry eye disease. Thus, this model enables study of the pathophysiological changes in dry eye and testing of therapeutic drug candidates for treating DED.

### Creating dry eye disease in an animal

In a first step of the process, scopolamine is administered as an anticholinergic agent, and benzalkonium chloride (BAC) is administered as an ocular irritant, to a rodent animal, such as a mouse or rat.

An anticholinergic agent is a substance that blocks the neurotransmitter acetylcholine in the central and/or peripheral nervous system. Examples of anticholinergic agents include, for example, scopolamine, scopolamine hydrochloride, scopolamine methobromide, atropine, atropine methyl nitrate, and atropine sulfate. In the methods disclosed herein, the anticholinergic agent is scopolamine or scopolamine hydrochloride.

In the disclosed methods, scopolamine is administered systemically. Methods for systemic administration include injection, transdermal patch, and implantation of a pump, such as an osmotic pump that allows for continuous dosing of laboratory animals. Such methods for systemic administration in animal models are known in the art. In specific embodiments, scopolamine is administered by osmotic mini-pump. Suitable osmotic mini-pumps include, for example, ALZET osmotic pumps (Durect Corporation, Cupertino, California).

To implant the pump, the rodent is anaesthetized and fitted with the pump subcutaneously. The pump provides a controlled amount of scopolamine. Exemplary concentrations of scopolamine include 0.1-4.0 mg per 20 g. body weight per day, 0.5-3.5 mg per 20 g. body weight per day, 1.0-3.0 mg per 20 g. body weight per day, or about 2.0 mg per 20 g. body weight per day. Scopolamine is administered for several days or several weeks, for example, 1, 2, 3, 4 weeks or more.

An ocular irritant is a substance that produces irritation in the eye. Examples of ocular irritants include surfectants, preservatives, allergens, fine particles, and dessicating agents or environmental conditions. In the methods disclosed herein, the ocular irritant is benzalkonium chloride (BAC).

In the disclosed methods, BAC is administered topically to the ocular surface. BAC can be administered at a concentration of 0.05-1.0%, and in specific embodiments, 0.1-0.2%, in a dosage of 0.5-2.0 µl per dose, for example 1 µl per dose. Administration can be one to four times per day, for example, twice daily, for one to three days per week. In some embodiments, administration is performed twice daily for two days per week. Administration of BAC can be performed for one or more weeks, for example, 1, 2, 3, 4 weeks or more.

### Evaluating dry eye conditions in model animals

Dry eye disease is confirmed in the model animal by detecting a reduction in tear production and an increase in ocular irritation, relative to control levels. Dry eye can further be confirmed by detecting an increase in angiogenesis and/or lymphangiogenesis, relative to control levels.

Throughout this disclosure, control levels represent the levels in an untreated rodent animal eye. Where an evaluation is directed to the efficacy of an agent being applied systemically, an untreated eye is an eye in an untreated rodent animal. An untreated animal can be naive (receiving essentially no treatment) or a sham operated animal (receiving an implanted pump that does not deliver an agent, or that delivers a placebo, such as saline, to the animal, or receiving a surgery without actual implantation of a pump). Where an agent is applied topically to the eye and not systemically, a control can also be the untreated eye in an animal with one treated eye. In the context of evaluating induction of dry eye conditions by administration of scopolamine and BAC, control levels are levels in eyes of untreated animals.

Reduced tear production is identified as a reduction in tear production of at least 10%, 20%, 30%, 40%, or 50% relative to control levels. Methods to measure tear production include the phenol red thread (PRT) test and Schirmer's test. In the PRT test, animals are restrained without anaesthesia and a phenol red loaded thread (such as ZONE-QUICK, FCI Ophthalmics, Pembroke, MA) is placed in the medial or lateral canthus (inner or outer corner of the eye), or contacting the conjunctival or lacrimal sac, for 20 seconds to measure tear production. The amount of thread that turns red (resulting from the change in pH from wetting by alkaline tears) is measured in millimeters. The procedure can then be repeated on the other eye to obtain 2 measures for each mouse. Schirmer's test uses paper strips to measure the production of tears. The test involves placing a small strip of filter paper inside the lower eyelid (conjunctival sac). The strip is maintained in place for several minutes. The paper is then removed and the amount of moisture is measured in mm.

Increased ocular irritation is identified by detecting, within the eye, one or more of: an increase in inflammatory cells, an increase in inflammatory cytokines in the eye, or an increase in fluorescein staining, relative to control levels.

An increase in inflammatory cells is an increase of at least 10%, 20%, 30%, 40%, or 50% of one or more types of inflammatory/immune cells, relative to control levels. Inflammatory/immune cell types include myeloid cells (including neutrophils, monocytes, eosinophils, and basophils) and lymphocytes (including T cells and B cells). Inflammatory cells can be detected and measured by various methods known in the art. Most commonly, inflammatory cells are measured by determining the presence/abundance of cells with identifying cell-surface markers. For example, both myeloid cells and lymphocytes are CD45+, and thus this marker can be used to identify the presence of inflammatory cells relative to non-inflammatory cells. Lymphocyte-specific markers include CD3 (identifying T cells), CD4 (identifying helper T cells), CD8 (identifying cytotoxic T cells), and B220 or CD19 (identifying B cells). Additional cell surface markers are known in the art, as are antibodies and other agents that detect these markers. Antibodies that detect these markers can be used to identify inflammatory cells, for example, by histological examination of corneal tissue and antibody staining of tissue sections. Antibodies that detect these markers can also be used by applying the antibodies to a population of cells to stain for expression of specific markers and processing the cells by flow cytometry to quantify cells expressing the markers.

An increase in inflammatory cytokines is an increase of at least 10%, 20%, 30%, 40%, or 50% of one or more types of inflammatory cytokines, relative to control levels. Inflammatory cytokines include interleukins (ILs) such as IL-1, IL-2, IL-3, IL-4, IL-5, IL-6, IL-7, IL-8, IL-9, IL-10, IL-11, IL-12, IL-13, tumor necrosis factors (TNFs) such as TNF-α, TNF-β, TGF-β (transforming growth factor-β), CXCL9, and interferons (IFNs) such as IFN-γ. Methods to measure inflammatory cytokines include immunoassays, such as an enzyme linked immunosorbent assay (ELISA), that use antibodies or other agents that detect cytokines to identify and quantify cytokines in a sample.

An increase in fluorescein staining is a score of greater than 5, 8, 10, or 12, using National Eye Institute (NEI) scoring. For fluorescein staining, sodium fluorescein is applied to the ocular surface of an animal, typically without sedation. Several minutes after application, corneal fluorescein staining is scored under a microscope using a blue light. Irregularities in the eye, such as abrasions and inflammation, fluoresce with greater intensity relative to healthy corneal tissue. Fluorescence is scored using the National Eye Institute (NEI) scoring standards. In NEI scoring, the five sectors of the cornea (central, superior, inferior, nasal and temporal) are scored individually on a scale of 0-3 for fluorescence, with 0 indicating no staining and 3 indicating extensive staining, with a maximal score of 15 per eye. If a different scoring standard or fluorescence technique is used, the increase in fluorescein staining would be identified as an increase of at least 10%, 20%, 30%, 40%, or 50% in fluorescence, relative to control levels.

Measures of corneal angiogenesis and/or lymphangiogenesis can further confirm dry eye. For studying angiogenesis and lymphangiogenesis, the animal is euthanized and the cornea is dissected and incubated with agents that identify new lymphatic vessel formation, such as antibodies to lymphatic vessel endothelial hyaluronan receptor 1 (LYVE1), or agents that identify new blood vessel formation, such as antibodies to CD31, an endothelial precursor cell marker. Identification of regions of new blood or lymphatic vessel growth can be used as an additional indicator of deteriorating ocular conditions and dry eye development.

### Testing candidate agents for dry eye disease

The disclosed rodents (rats and mice) are useful for the study of DED and testing of candidate agents for the treatment of dry eye.

Accordingly, disclosed herein are methods of testing the efficacy of a candidate agent for the treatment of dry eye, involving administering a candidate agent to an animal with DED induced by the disclosed methods, and testing the ability of the candidate to treat DED.

By "treating" it is meant ameliorating the symptoms or conditions of DED, or preventing DED (e.g., preventing or delaying the onset, or limiting the development of DED).

The terms "test agent" and "candidate agent" are used interchangeably herein to refer to any agent contemplated for the treatment of dry eye. The agent can be a recombinant, modified or natural nucleic acid molecule; a synthetic, modified or natural peptide; a synthetic, modified or natural polypeptide, including antibodies; or an organic or inorganic compound, including small molecules. The term "small molecule" refers to compounds having a molecular mass of less than 3000 Daltons, preferably less than 2000 or 1500, more preferably less than 1000 or 800 Daltons.

The test agent to be evaluated can be administered to the animal topically or systemically. For example, the agent can be administered in the form of an eye drop or an intranasal spray. Alternatively, the agent can be administered orally or parenterally and, if administered parenterally, can be in the form of solution, suspension, ointment, injection, suppository and the like. The suitable dose of the candidate agent can be decided based on the body weight of the animal and route of administration. Conditions for administration, such as timing and number of times thereof, are also decided depending on the agent to be used.

### Administration of a candidate agent at the start of DED induction

In one embodiment, the candidate agent is administered coincident with the start of DED induction. In some embodiments, sets of rodent animals to be tested are randomized to the following groups: naive (untreated), sham (systemic administration of saline, implantation of pump but no scopolamine treatment, or surgery analogous to pump implantation without actual implantation of a pump), benzalkonium chloride plus scopolamine (BAC plus Scop), test agent alone, or test agent plus BAC plus Scop. Animals in the test agent groups are administered the candidate agent coincident with the start of dry eye induction, that is, starting the same day or during the same week as DED induction begins. All treatments are performed for at least one, two, three, or four weeks. Tear production and corneal fluorescein staining are measured on days 7, 14, 21, and 28. On day 28, animals are euthanized and the extraorbital lacrimal gland and cornea can be harvested for further study.

The ability of the candidate agent to prevent, delay the onset of, or limit the development of DED is determined by detecting the ability of the candidate agent to maintain tear production, and/or prevent or limit ocular irritation, at similar levels to control levels. In this context, controls levels are levels of an untreated eye (i.e., eye without the treatment of the candidate agent), which can include an eye in animals receiving scopolamine and benzalkonium chloride but not receiving the candidate agent, or one of the eyes in an animal receiving scopolamine and benzalkonium chloride as well as the candidate agent wherein the candidate agent is being applied topically to the other eye of the same animal.

In one embodiment, animals receiving the test agent plus BAC plus Scop do not develop DED, that is, tear production is maintained at or within 5%, 10%, or 15% of control levels throughout the study, and/or levels of ocular inflammation, as measured by the levels of inflammatory cells, levels of inflammatory cytokines in the eye, and/or fluorescein staining score, are maintained at or within 5%, 10%, or 15% of control levels, throughout the study.

In another embodiment, animals receiving the test agent plus BAC plus Scop show a delay in the onset of DED symptoms, that is, there is a delay in the development of reduced tear production and/or ocular irritation by one, two, or three weeks, relative to animals receiving BAC plus Scop but not receiving the candidate agent.

In another embodiment, animals receiving the test agent plus BAC plus Scop develop milder DED symptoms, that is, reduced tear production and/or ocular irritation occur in these animals, but the reduced tear production and/or ocular irritation occur at levels intermediate between levels in animals without receiving DED inducing treatment (e.g, naive animals) and levels observed in animals receiving BAC plus Scop but not receiving the test agent.

### Administration of a candidate agent subsequent to DED induction

In another embodiment, the candidate agent is administered after the start of DED induction. In some embodiments, animals in the test agent groups are administered the candidate agent starting one to two weeks after dry eye induction begins, i.e., between days 7-14 after the start of dry eye induction. The test agent is administered for one to three weeks. Tear production and corneal fluorescein staining are measured on days 7, 14, 21, and 28 from the start of dry eye induction. On day 28, animals are euthanized and the extraorbital lacrimal gland and cornea can be harvested for further study.

The ability of the candidate agent to treat DED is determined by detecting an increase in tear production, and/or a decrease in ocular irritation in animals receiving the candidate agent and BAC plus Scop, relative to control levels which, in this context, can be levels in eyes of animals receiving BAC plus Scop but not receiving the candidate agent.

### EXAMPLES

### Example 1. Creating dry eye conditions in an animal.

***Animals.*** All animal related procedures were reviewed and approved by the Regeneron IACUC, and conducted in compliance to the ARVO Statement for the Use of Animals in Ophthalmic and Vision Research. Adult C57BL/6 mice (8-10 weeks old) were purchased from Taconic Biosciences (Germantown, NY).

***Dry eye model induction*:** 27 adult, male C57BL/6 mice were randomized to naive (untreated), sham (receiving surgery equivalent to surgery for implantation of pump, but without actual implantation of pump and without scopolamine or BAC treatment), benzalkonium chloride (BAC), scopolamine (Scop) or BAC plus Scop groups.

***Implantation of scopolamine pump.*** For Scop group, mice were subcutaneously implanted with an osmotic pump filled with Scop (2 mg/20 g body weight/day) lasting for 4 weeks. Mice were anesthetized with ketamine (120 mg/kg, IP) and xylazine (5 mg/kg, IP). Using aseptic microsurgical technique and an operating microscope, a mini-osmotic pump was placed beneath the back skin, then the skin was sutured, and erythromycin ophthalmic ointment was applied to the wound to prevent infection.

***Benzalkonium chloride treatment.*** For BAC treatment, 1 µl of 0.1-0.2% BAC was topically administrated on the right eye ocular surface, twice daily, 2 days per week, for four weeks.

***Tear production measurement and corneal fluorescein staining score.*** Tear production and corneal fluorescein staining were performed once per week, on day 7, 14, 21, and 28. For tear production measurement, with the animal restrained, phenol red thread was put on the eye temporal sac (without touching the cornea) and held in place for 1 minute. The wet portion of the thread was scaled per mm as tear production. For fluorescein staining, 0.5 µl of 2% sodium fluorescein was put on the ocular surface. Five minutes after application, corneal fluorescein staining was scored under a microscope using the National Eye Institute (NEI) scoring standards. For scoring, the cornea is divided into five sectors (central, superior, inferior, nasal and temporal), each of which is scored on a scale of 0-3 for fluorescence, with 0 indicating no staining and 3 indicating extensive staining, with a maximal score of 15 per eye.

***Quantification of corneal angiogenesis, lymphangiogenesis and lacrimal gland infiltration.*** On day 28 after induction of dry eye, under deep ketamine (120 mg/kg, IP) and xylazine (5 mg/kg, IP) anesthesia, eyeballs were collected in 4% paraformaldehyde (PFA) for corneal angiogenesis and lymphangiogenesis studies. The extraorbital lacrimal glands were harvested for flow cytometry, to check for immune cell infiltration.

***Histological Analysis.*** The cornea was dissected from the eyeballs, washed in PBS, blocked for 1 hour at room temperature, with 10% normal donkey serum and 1% Triton X-100 in PBS to block antibody non-specific sites. Then the samples were washed in PBS, incubated with a cocktail of antibodies to lymphatic vessel endothelial hyaluronan receptor 1 (LYVE1) (Regeneron Pharmaceuticals, Inc., Tarrytown, NY) at 1:1000, and antibodies to CD31 (Santa Cruz Biotechnology, Santa Cruz, CA) at 1:100 at 4°C overnight. Samples were then washed in phosphate buffered saline (PBS) and incubated with secondary antibody conjugated dye, AlexaFluor 488 donkey anti rat IgG(H+L)1:100 (Life Technologies, A21208, Graceland, NY) and AlexaFluor 594 donkey anti rat IgG(H+L)1:400 (Life Technologies, A21207) for 2 hours at room temperature. After incubation, samples were washed in PBS and flat-mounted onto glass slides. Images of stained blood and lymphatic vessels were captured using a digital RT SE Spot camera attached to a fluorescence microscope (Nikon Eclipse 80i). Image software was used for image analysis.

***Flow cytometry.*** Extraorbital lacrimal glands were placed in PBS (Life Technologies) with 3% fetal bovine serum solution (FBS) (Life Technologies) after dissection. The glands were dissociated with MACS gentle dissociator (Miltenyi Biotech, San Diego, CA) program spleen-04. Cell suspension was filtered through a 70 um strainer (Miltenyi Biotech, San Diego, CA) to remove tissue debris. Cells were washed once with FACS buffer (PBS with 3% FBS and 2 mM EDTA) (Life Technologies). Washed cells were resuspend in FACS buffer with FcR blocking Ab (1:50 dilution) (Biolegend, San Diego, CA) and incubated for 15 minutes at 4°C. Cells were spun down to remove FcR blocking Ab and further stained with LIVE/DEAD Fixable Blue Dead Cell Stain Kit (1:500 dilution) (Life Technologies) together with CD45 PerCP-Cy5.5, Clone 30-F11, Rat IgG2b (1:100 dilution) (BD Bioscience). The cells were stained for 25 min at 4°C followed by two washes with FACS buffer. The cells were then resuspend in Stabilizing Fixative (BD) and acquired on LSR II (BD Bioscience) following standard protocol provided by the company. Data was analyzed by FlowJo (Tree Star, Ashland, OR).

***Statistical Analysis.*** Statistical analyses of parametric data were performed using two-way ANOVA and Tukey's multiple comparison test (performed for corneal vessel length, lymphatic vessel length studies only) with GraphPad Prism (version 6.0a, GraphPad Software Inc., San Diego, CA). Data are shown as means ± standard error of the mean (SEM). A p-value of less than 0.05 was considered as statistically significant. * =*p*<*0.05;* ** = *p*<*0.01;* *** =*p*<*0.001;* **** = *p<0.0001*.

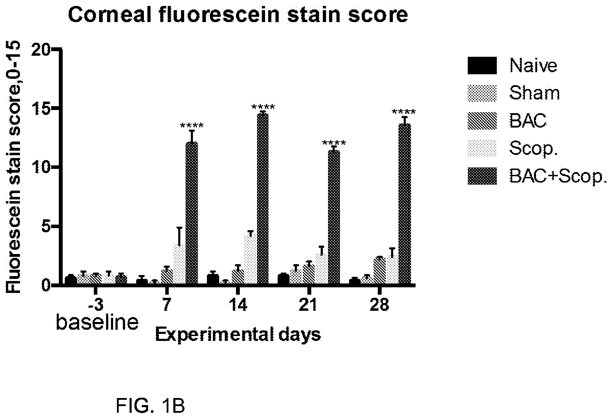

***Results.*** As shown in FIG. 1A, post-systemic delivery of Scop. plus topical administration of BAC, tear production significantly decreased. In addition, corneal stain score markedly increased (FIG. 1B). The change was evident after one week of Scop + Bac administration.

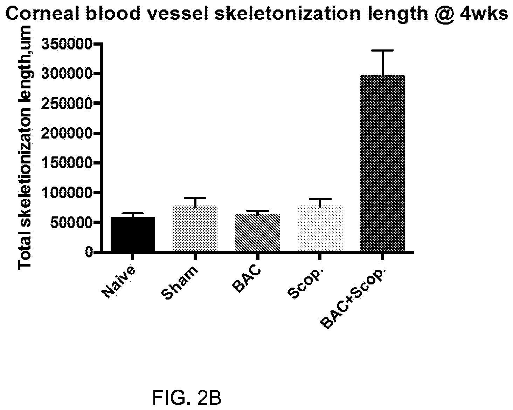

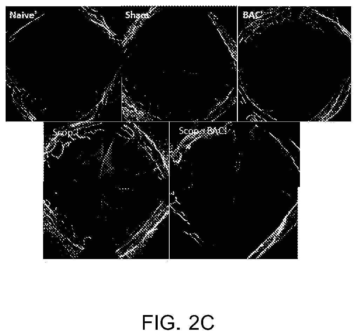

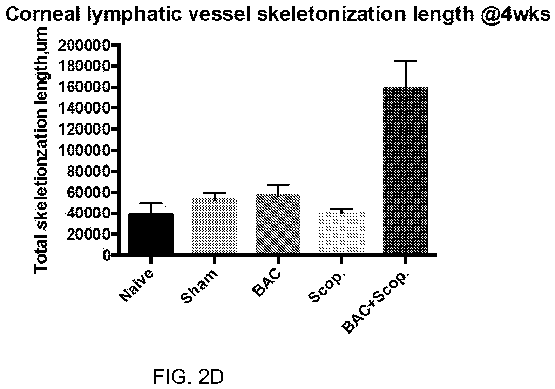

Additionally, as depicted in FIGS. 2A and 2B, corneal blood vessel skeletonization length significantly increased after 4 weeks of treatment with BAC + Scop. Similarly, as demonstrated in FIGS. 2C and 2D, corneal lymphatic vessel skeletonization length increased after 4 weeks of BAC +Scop treatment.

Moreover, extraorbital lacrimal glands contained immune cell infiltrate after 4 weeks of treatment, as indicated in FIG. 3.

### Example 2. Testing ability of candidate agent to prevent or inhibit onset of dry eye

For studies of the efficacy of candidate agents to prevent or inhibit the development of dry eye, sets of mice are randomized to the following groups: naive (untreated), sham (receiving surgery equivalent to implantation of pump, but no actual implantation of pump and without scopolamine or BAC treatment), benzalkonium chloride plus scopolamine (BAC plus Scop), test agent alone, or test agent plus BAC plus Scop.

Dry eye is induced as described above. For BAC plus Scop treatment, mice are subcutaneously implanted with an osmotic pump delivering scopolamine (2 mg/20 g body weight/day), and 1 µl of 0.1-0.2% BAC is topically administrated on the right eye ocular surface, twice daily, 2 days per week.

Animals in the test agent groups are administered the candidate agent starting on the same day dry eye induction begins. The candidate agent is administered to the animal in the form of an eye drop, an intranasal spray, or parenterally in the form of a solution for injection. The suitable dose of the candidate agent and frequency of administration is decided based on the body weight of the animal and route of administration. Timing and frequency of administration depend on the agent to be used. All treatments are performed for at least one, two, three, or four weeks. Tear production and corneal fluorescein staining are measured on days 7, 14, 21, and 28. On day 28, animals are euthanized and the extraorbital lacrimal gland and cornea are harvested for flow cytometry and histology. Corneal angiogenesis and lymphangiogenesis are also quantified.

The efficacy of the candidate agent is determined by detecting the ability of the candidate agent to maintain tear production, and/or prevent or limit ocular irritation, relative to control levels, e.g., levels in an eye of animals receiving scopolamine and benzalkonium chloride but not receiving the candidate agent.

### Example 3. Testing ability of candidate agent to treat dry eye condition

For studies of efficacy of candidate agents to treat dry eye, sets of mice are randomized to the following groups: naive (untreated), sham (receiving surgery equivalent toimplantation of pump, but without actual implantation of pump and without scopolamine or BAC treatment), benzalkonium chloride plus scopolamine (BAC plus Scop), test agent alone, or test agent plus BAC plus Scop.

Dry eye is induced as described above. For BAC plus Scop treatment, mice are subcutaneously implanted with an osmotic pump delivering scopolamine (2 mg/20 g body weight/day), and 1 µl of 0.1-0.2% BAC is topically administrated on the right eye ocular surface, twice daily, 2 days per week. Dry eye induction methods continue for at least one, two, three, or four weeks.

Animals in the test agent groups are administered the candidate agent starting one to two weeks after dry eye induction begins, e.g., between days 7-14 after the start of dry eye induction. The candidate agent is administered to the animal in the form of an eye drop, an intranasal spray, or parenterally in the form of a solution for injection. The suitable dose of the candidate agent and frequency of administration is decided based on the body weight of the animal and route of administration. Timing and frequency of administration depend on the agent to be used. The test agent is administered for one to three weeks. Tear production and corneal fluorescein staining are measured on days 7, 14, 21, and 28 from the start of dry eye induction. On day 28, animals are euthanized and the extraorbital lacrimal gland and cornea are harvested for flow cytometry and histology. Corneal angiogenesis and lymphangiogenesis are also quantified.

Efficacy is determined by detecting an increase in tear production and/or a decrease in ocular irritation in animals receiving the candidate agent and BAC plus Scop, relative to control levels (e.g., levels in an eye of animals receiving BAC plus Scop but not receiving the candidate agent).

## Claims

1. A method of inducing dry eye disease in a rodent animal, comprising administration of scopolamine and benzalkonium chloride to said rodent animal.

2. The method of claim 1, wherein the rodent animal is a mouse or rat.

3. The method of claim 1, wherein scopolamine is administered by implantation of an infusion pump.

4. The method of claim 3, wherein scopolamine is administered at a concentration of 0.4-4.0 mg per 20 g. body weight per day.

5. The method of claim 1, wherein benzalkonium chloride is administered topically to the ocular surface of said rodent animal.

6. The method of claim 5, wherein benzalkonium chloride is administered at a concentration of 0.05-0.4%, one to four times per day.

7. The method of any of claims 1-5, wherein scopolamine and benzalkonium chloride are administered to said rodent animal for a period of up to two months.

8. The method of claim 6, wherein scopolamine and benzalkonium chloride are administered to said rodent animal for two to four weeks.

9. The method of claim 8, wherein the rodent is an 8-10 week old C57BL/6 male mouse, the scopolamine is administered by a subcutaneously implanted osmotic pump at a concentration of 2 mg per 20 g body weight per day for four weeks, and benzalkonium chloride is administered topically on an ocular surface at a concentration of 0.2%, twice per day, two days per week for four weeks.

10. The method of claim 1, wherein said dry eye condition is **characterized by** reduced tear production, and increased ocular irritation, and optionally further **characterized by** increased angiogenesis and/or increased lymphangiogenesis in a treated eye, relative to control levels, optionally wherein reduced tear production is detected by detecting a reduction in tear production of at least 10% relative to control levels.

11. The method of claim 10, wherein the increased ocular irritation is measured by detecting, within the eye, one or more of: an increase in inflammatory cells, an increase in inflammatory cytokines in the eye, or an increase in fluorescein staining, relative to control levels.

12. Scopolamine and benzalkonium chloride for use in a method of testing the efficacy of a candidate agent for the treatment of dry eye, wherein the scopolamine and benzalkonium chloride are administered to a rodent animal to induce dry eye in said rodent animal, and said candidate agent is subsequently administered to said rodent animal, wherein the efficacy of said candidate agent is determined by detecting an increase in tear production of at least 10% and/or a decrease in ocular irritation of at least 10%, relative to animals receiving scopolamine and benzalkonium chloride but not receiving the candidate agent.

13. The scopolamine and benzalkonium chloride for use of claim 12, wherein:
(i) said candidate agent is administered at least 7 days after the start of administration of scopolamine and benzalkonium chloride;
(ii) said candidate agent is administered at least 14 days after the start of administration of scopolamine and benzalkonium chloride;
(iii) said candidate agent is administered at least 28 days after the start of administration of scopolamine and benzalkonium chloride; or
(iv) said candidate agent is administered topically or systemically, optionally wherein said topical administration is administration of the candidate agent in a drop, spray, or gel form to the eye or nose of said rodent animal, or optionally wherein said systemic administration is selected from oral administration, injection, or infusion.

14. The scopolamine and benzalkonium chloride for use of claim 12, wherein said candidate agent is administered concurrently with the start of administration of scopolamine and benzalkonium chloride, optionally wherein the efficacy of said candidate agent is determined based on the ability of the candidate agent to limit or eliminate reduction in tear production, and/or limit or eliminate increased ocular irritation, relative to animals receiving scopolamine and benzalkonium chloride but not receiving the candidate agent.

## Patentansprüche

1. Verfahren zum Hervorrufen einer Trockenaugenerkrankung bei einem Nagetier, umfassend das Verabreichen von Scopolamin und Benzalkoniumchlorid an das Nagetier.

2. Verfahren nach Anspruch 1, wobei das Nagetier eine Maus oder Ratte ist.

3. Verfahren nach Anspruch 1, wobei Scopolamin durch Implantation einer Infusionspumpe verabreicht wird.

4. Verfahren nach Anspruch 3, wobei Scopolamin mit einer Konzentration von 0,4 bis 4,0 mg pro 20 g. Körpergewicht pro Tag verabreicht wird.

5. Verfahren nach Anspruch 1, wobei Benzalkoniumchlorid an die Augenoberfläche des Nagetiers topisch verabreicht wird.

6. Verfahren nach Anspruch 5, wobei Benzalkoniumchlorid mit einer Konzentration von 0,05 bis 0,4 % einmal bis viermal pro Tag verabreicht wird.

7. Verfahren nach einem der Ansprüche 1 bis 5, wobei Scopolamin und Benzalkoniumchlorid für einen Zeitraum von bis zu zwei Monaten dem Nagetier verabreicht werden.

8. Verfahren nach Anspruch 6, wobei Scopolamin und Benzalkoniumchlorid für zwei bis vier Wochen dem Nagetier verabreicht werden.

9. Verfahren nach Anspruch 8, wobei das Nagetier eine 8 bis 10 Wochen alte männliche C57BL/6-Maus ist, das Scopolamin durch eine subkutan implantierte osmotische Pumpe mit einer Konzentration von 2 mg pro 20 g Körpergewicht pro Tag für vier Wochen verabreicht wird und Benzalkoniumchlorid auf einer Augenoberfläche mit einer Konzentration von 0,2 % zweimal pro Tag zwei Tage pro Woche für vier Wochen topisch verabreicht wird.

10. Verfahren nach Anspruch 1, wobei die Trockenaugenerkrankung durch reduzierte Tränenproduktion und erhöhte Augenirritation gekennzeichnet ist und optional ferner durch erhöhte Angiogenese und/oder erhöhte Lymphangiogenese in einem behandelten Auge relativ zu Kontrollebenen gekennzeichnet ist, wobei optional eine reduzierte Tränenproduktion durch Nachweisen einer Reduzierung in der Tränenproduktion von mindestens 10 % relativ zu Kontrollebenen nachgewiesen wird.

11. Verfahren nach Anspruch 10, wobei die erhöhte Augenirritation gemessen wird durch Nachweisen, innerhalb des Auges, von einem oder mehreren von: einer Zunahme in entzündlichen Zellen, einer Zunahme in inflammatorischen Zytokinen in dem Auge oder einer Zunahme in der Fluoreszeinverfärbung relativ zu Kontrollebenen.

12. Scopolamin und Benzalkoniumchlorid zur Verwendung in einem Verfahren zum Testen der Effizienz eines Kandidatenagens zur Behandlung eines trockenen Auges, wobei das Scopolamin und Benzalkoniumchlorid einem Nagetier verabreicht werden, um ein trockenes Auge bei dem Nagetier hervorzurufen, und das Kandidatenagens anschließend dem Nagetier verabreicht wird, wobei die Effizienz des Kandidatenagens durch Nachweisen einer Zunahme in der Tränenproduktion von mindestens 10 % und/oder einer Abnahme in der Augenirritation von mindestens 10 % relativ zu Tieren, die Scopolamin und Benzalkoniumchlorid erhalten, das Kandidatenagens aber nicht erhalten, bestimmt wird.

13. Scopolamin und Benzalkoniumchlorid zur Verwendung nach Anspruch 12, wobei:
(i) das Kandidatenagens mindestens 7 Tage nach dem Beginn der Verabreichung von Scopolamin und Benzalkoniumchlorid verabreicht wird;
(ii) das Kandidatenagens mindestens 14 Tage nach dem Beginn der Verabreichung von Scopolamin und Benzalkoniumchlorid verabreicht wird;
(iii) das Kandidatenagens mindestens 28 Tage nach dem Beginn der Verabreichung von Scopolamin und Benzalkoniumchlorid verabreicht wird; oder
(iv) das Kandidatenagens oberflächlich oder systemisch verabreicht wird, wobei optional die topische Verabreichung die Verabreichung des Kandidatenagens in einer Tropfen-, Sprüh- oder Gelform an das Auge oder die Nase des Nagetiers ist, oder wobei optional die systemische Verabreichung aus oraler Verabreichung, Injektion oder Infusion ausgewählt wird.

14. Scopolamin und Benzalkoniumchlorid zur Verwendung nach Anspruch 12, wobei das Kandidatenagens gleichzeitig mit dem Beginn der Verabreichung von Scopolamin und Benzalkoniumchlorid verabreicht wird, wobei optional die Effizienz des Kandidatenagens basierend auf der Fähigkeit des Kandidatenagens, die Tränenproduktion zu begrenzen oder zu eliminieren, und/oder eine erhöhte Augenirritation relativ zu Tieren zu begrenzen oder zu eliminieren, die Scopolamin und Benzalkoniumchlorid erhalten, aber nicht das Kandidatenagens erhalten, bestimmt wird.

## Revendications

1. Procédé d'induction d'une sécheresse oculaire chez un animal rongeur, comprenant l'administration de scopolamine et de chlorure de benzalkonium au dit animal de rongeur.

2. Procédé selon la revendication 1, dans lequel l'animal rongeur est une souris ou un rat.

3. Procédé selon la revendication 1, dans lequel la scopolamine est administrée par l'implantation d'une pompe à perfusion.

4. Procédé selon la revendication 3, dans lequel la scopolamine est administrée à une concentration de 0,4-4,0 mg par 20 g. de poids corporel par jour.

5. Procédé selon la revendication 1, dans lequel le chlorure de benzalkonium est administré par voie topique à la surface oculaire dudit animal rongeur.

6. Procédé selon la revendication 5, dans lequel le chlorure de benzalkonium est administré à une concentration de 0,05-0,4%, un à quatre fois par jour.

7. Procédé selon l'une quelconque des revendications 1 à 5, dans lequel la scopolamine et le chlorure de benzalkonium sont administrés au dit animal rongeur durant une période allant jusqu'à deux mois.

8. Procédé selon la revendication 6, dans lequel la scopolamine et le chlorure de benzalkonium sont administrés au dit animal rongeur durant deux à quatre semaines.

9. Procédé selon la revendication 8, dans lequel le rongeur est une souris mâle C57BL/6 âgée de 8 à 10 semaines, la scopolamine est administrée par une pompe osmotique implantée par voie sous-cutanée à une concentration de 2 mg par 20 g de poids corporel par jour durant quatre semaines, et le chlorure de benzalkonium est administré par voie topique sur une surface oculaire à une concentration de 0,2 %, deux fois par jour, deux jours par semaine durant quatre semaines.

10. Procédé selon la revendication 1, dans lequel ladite sécheresse oculaire est **caractérisée par** une production lacrymale réduite, et une irritation oculaire accrue, et éventuellement **caractérisée en outre par** une angiogenèse accrue et/ou une lymphangiogenèse accrue dans un oeil traité, par rapport aux niveaux de contrôle, éventuellement dans lequel la production lacrymale réduite est détectée par la détection d'une réduction de la production lacrymale d'au moins 10 % par rapport aux niveaux de contrôle.

11. Procédé selon la revendication 10, dans lequel l'irritation oculaire accrue est mesurée par la détection, dans l'oeil, d'une ou plusieurs parmi : une augmentation des cellules inflammatoires, une augmentation des cytokines inflammatoires dans l'oeil, ou une augmentation de la coloration fluorescéine, par rapport aux niveaux de contrôle.

12. Scopolamine et chlorure de benzalkonium à utiliser dans un procédé de test de l'efficacité d'un agent candidat pour le traitement de la sécheresse oculaire, dans lesquels la scopolamine et le chlorure de benzalkonium sont administrés à un animal rongeur pour induire une sécheresse oculaire chez ledit animal rongeur, dans lesquels l'efficacité dudit agent candidat est déterminé par la détection d'une augmentation de la production lacrymale d'au moins 10 % et/ou d'une diminution de l'irritation oculaire d'au moins 10 %, par rapport aux animaux recevant de la scopolamine et du chlorure de benzalkonium mais ne recevant pas l'agent candidat.

13. Scopolamine et chlorure de benzalkonium à utiliser selon la revendication 12, dans lesquels :
(i) ledit agent candidat est administré au moins 7 jours après le début de l'administration de la scopolamine et du chlorure de benzalkonium ; ou
(ii) ledit agent candidat est administré au moins 14 jours après le début de l'administration de la scopolamine et du chlorure de benzalkonium ; ou
(iii) ledit agent candidat est administré au moins 28 jours après le début de l'administration de la scopolamine et du chlorure de benzalkonium ; ou
(iv) ledit agent candidat est administré par voie topique ou systémique, éventuellement dans lequel ladite administration topique est l'administration de l'agent candidat sous forme de goutte, de pulvérisation ou sous forme de gel dans l'oeil ou dans le nez dudit animal rongeur, ou éventuellement, dans lequel ladite administration systémique est choisie parmi l'administration orale, l'injection ou la perfusion.

14. Scopolamine et chlorure de benzalkonium à utiliser selon la revendication 12, dans lesquels ledit agent candidat est administré simultanément avec le début de l'administration de la scopolamine et du chlorure de benzalkonium, éventuellement dans lequel l'efficacité dudit agent candidat est déterminée sur base de la capacité de l'agent candidat à limiter ou éliminer la réduction de la production lacrymale, et/ou à limiter ou éliminer une irritation oculaire accrue, par rapport aux animaux recevant de la scopolamine et du chlorure de benzalkonium, mais ne recevant pas l'agent candidat.
